Europäisches Patentamt

(19) European Patent Office

Office européen des brevets

(11) Publication number : **0 463 999 A1**

# (12) EUROPEAN PATENT APPLICATION

(21) Application number : **91810491.0**

(22) Date of filing : **24.06.91**

(51) Int. Cl.$^5$ : **A01H 1/02, A01H 1/06**

(30) Priority : **25.06.90 GB 9014090**

(43) Date of publication of application :
**02.01.92 Bulletin 92/01**

(84) Designated Contracting States :
**AT BE CH DE DK ES FR GB GR IT LI LU NL SE**

(71) Applicant : **SANDOZ LTD.**
**Lichtstrasse 35**
**CH-4002 Basel (CH)**
(84) **BE CH DK ES FR GB GR IT LI LU NL SE**

(71) Applicant : **SANDOZ-PATENT-GMBH**
**Humboldtstrasse 3**
**W-7850 Lörrach (DE)**
(84) **DE**

(71) Applicant : **SANDOZ-ERFINDUNGEN**
**Verwaltungsgesellschaft m.b.H.**
**Brunner Strasse 59**
**A-1235 Wien (AT)**
(84) **AT**

(72) Inventor : **Kootstra, Jille Jan**
**Jan Gooskaai 61**
**NL-1602 GC Enkhuizen (NL)**
Inventor : **Van Holst, Gerrit-Jan**
**De Gouw 8**
**NL-1602 DN Enkhuizen (NL)**

(54) Improvements in or relating to organic compounds.

(57)    Plants selected from Solanaceae, Asteraceae, Violaceae, Cucurbitaceae, Balsaminaceae, Begoniaceae, Geraniaceae and Primulaceae having homozygously or heterozygously the tolerance gene against a purine analogue and a method of selecting plants for male sterility comprising treating seedlings having homozygously the tolerance gene against a purine analogue with a purine analogue.

EP 0 463 999 A1

The invention relates to plants having a selectable form of genic male sterility. The invention provides male sterile Solanaceae, Asteraceae, Violaceae, Cucurbitaceae, Balsaminaceae, Begoniaceae, Geraniaceae and Primulaceae tolerant to purine analogues such as 2,6-diaminopurine.

Genic male sterile plants are useful in hybrid seed production.

Commercial F1 hybrid cultivars have become increasingly important in food and ornamental crops since the growing interest of breeders in hybrid vigour. In order to produce hybrid seed economically, the female parent must be prevented from self-fertilisation. This can be done by hand emasculation, chemical emasculation or by making use of genetic male sterility. Since hand emasculation is time consuming and expensive and chemical emasculation has been shown to be unreliable, there is now great interest in genetic male sterility.

There are three forms of genetic male sterility:

**1. Genic male sterility.**

Genic male sterility is usually monogenic recessive. Fertility in the hybrid can be restored by crossing male sterile plants with plants of a restorer line, which is homozygous male fertile. It is not possible to maintain a pure breeding male sterile line. This means that in seed production plots half of the plants, which are heterozygous male fertile, have to be removed.

**2. Cytoplasmic male sterility.**

Cytoplasmic male sterility is based solely on plasma genes transmitted maternally. Since the fertility in the hybrid cannot be restored it is only useful in plants where the hybrids do not need to set seed.

**3. Gene-Cytoplasmic male sterility**

This type of male sterility has found large scale application in the production of hybrid seed, since not only the fertility in the hybrid is restored by crossing with a male fertile restorer line, but also a pure breeding male sterile line can be maintained.

B. Moffatt et al reported in Plant Physiol. 86, 1150-1154 (1988) the chemical induction of Adenine Phosphoribosyl Transferase (APRT) deficiency in Arabidopsis thaliana (family of the Brassicaceae) by treatment of seeds with ethylmethane sulfonate and the selection of the mutants by germination of M2 seeds on a medium solidified with agar containing 2,6-diaminopurine. Only the mutated seeds that were homozygous APRT deficient were able to germinate and develop roots under these conditions and these plants appeared to be male sterile.

It has now surprisingly been found that plants selected from Solanaceae, Asteraceae, Violaceae, Cucurbitaceae, Balsaminaceae, Begoniaceae, Geraniaceae and Primulaceae which differ significantly from Brassicaceae in their way of flowering, can also be rendered tolerant to purine analogues by mutagenesis and that homozygous genic male sterile plants can be obtained by selfing thus obtained heterozygous plants having such recessive trait for resistance to purine analogues. The trait is presumably monogenic. The homozygous genic male sterile plants can be easily selected from the male fertile plants in the seedling stage. The male sterile plants will grow true leaves when sprayed with purine analogues, while the fertile plants do not.

It is particularly surprising that plants containing more than one isoenzyme of the APRT such as tomato [Burch and Stuchbury, Phytochemistry 25, 2445-2449 (1986)] can be made tolerant to purine analogues and have also acquired genetic male sterility.

The invention therefore provides purine analogue tolerant plants selected from Solanaceae, Asteraceae, Violaceae, Cucurbitaceae, Balsaminaceae, Begoniaceae, Geraniaceae and Primulaceae. Examples of suitable Solanaceae include pepper (Capsicum spec.) tomato, aubergine (Solanum melongena L.), petunia, Schizanthus, Salpiglossis, Exacum, Datura, Nicotiania, Biowallia and Brunfelvia plants. Typical examples of Asteraceae include chicory (Cichorium intybus L.) and endive (Cichorium endivia L.). A typical representative of Violaceae suitable for use according to the invention, is violet (Viola). A typical representative of Cucurbitaceae suitable for use according to the invention is melon (Cucumis melo L.). A typical representative of Balsaminaceae suitable for use according to the invention is impatiens. A typical representative of Begoniaceae suitable for use according to the invention is begonia. A typical representative of Geraniacea suitable for use according to the invention is geranium (Pelargonium hortorum). A typical representative of Primulaceae suitable for use according to the invention is cyclamen.

The invention also provides a method of selecting plants having homozygously the tolerance gene against a purine analogue, which comprises treating the seedlings during the development of the first pair of true leaves with a plant growth regulating amount of a purine analogue and selecting the plants which are not affected by such treatment.

The thus obtained plants are homozygous male sterile. They can be used in a breeding program for the production of hybrid seeds. The method of the invention is particularly suitable for plants of the familles Solanaceae, Asteraceae, Violaceae, Cucurbitaceae, Balsaminaceae, Begoniaceae, Geraniaceae and Primulaceae.

The seedlings employed as starting material in the method of the invention are obtained by inducing mutation in plants, and selfing the thus obtained heterozygous mature plants having a recessive trait for male sterility.

The mutagenesis of plants to heterozygous plants having a recessive trait for male sterility can be effected by any mutagenesis process known in the art, e.g. by somaclonal variation, irradiation etc. According to a preferred process, purine analogue resistant plants according to the invention are obtained by treatment of their seeds with a mutagenically effective amount of a mutagen such as ethylmethane sulfonate (EMS). The treatment with EMS is conveniently effected with an aqueous solution in a manner known for the mutagenesis of cells. The concentration of EMS to be employed will conveniently lie in the range of 0.01 to 3 % (v/v). The duration of the EMS treatment may vary within wide ranges e.g. between 2 and 20 hours; in general a treatment of 10 to 20 hours, e.g. of 14-16 hours will give satisfactory results.

The EMS treatment will conveniently be effected at room temperature, i.e. between 15 and 25° Centigrade.

The EMS treated seeds are then washed with water to remove the EMS, grown and the mature plants self-fertilized. The homozygous male sterile plants may be selected by treatment of the seedlings of the self-fertilized plants with a purine analogue during the development of the first pair of true leaves: the homozygous male sterile plants according to the invention are not affected by such treatment, whereas the male fertile plants which are not tolerant to the treatment by a purine analogue will not develop their first true leaves and remain blind during such treatment.

The term purine analogue as used herein refers to a substance which differs chemically fron the naturally occurring purines in a limited number of structural features and exhibits antagonism to one or more of the natural purines in at least on biological system.

Purine analogues particularly suitable for the selection of male sterile plants according to the invention are 2,6-diaminopurine, 2-fluoroadenine, 6-mercaptopurine, 6-chloropurine, 6-fluoropurine, 6-methylpurine, 6-hydroxylaminopurine, 8-azapurine and 8-azaadenine, preferably 2,6-diaminopurine.

The purine analogues are preferably applied as a foliar aqueous spray. The amount to be applied is a plant growth regulating amount. Such amount can easily be determined by routine test with corresponding seedlings from non-mutated plants.

In general suitable spray liquors comprise from 0.1 to 15 mM, e.g. 3mM purine analogue per litre.

The spray application may be effected one or more times, each time conveniently till near the run off. In general one spray application - till near the run off - per day for up to 15 days, e.g. for 5 to 15 consecutive days will allow the desired selection.

The seddlings of which the development of the first pair of true leaves is not impaires can be grown under normal frowing conditions to maturity and are male sterile. Thus obtained homozygous male sterile plants according to the invention, can be crossed with male fertile plants to give male fertile heterozygous plants which are not tolerant to purine analogues.

It will be appreciated that homozygous male sterile plants have to be maintained for hydrid seed production. This can be achieved by pollinating a plant containing at least one allele for the tolerance to purine analogues with pollen from a plant which also carries at least one such allele, treating the seedlings thereof during the development of the first pair of true leaves with a purine analogue and selecting the plants which are not affected by such treatment.

The invention has the great advantage that it allows selection of male sterile plants before planting out in the field; roguing of heterozygous male fertile plants in seed production plots is accordingly no longer necessary with this form of genic male sterility.

The following example illustrates the invention.

## EXAMPLE

10000 Pepper seeds are mutagenized with 0.3 % (v/v) ethylmethane sulfonate during 14-16 hours at room temperature. After thorough washing with water, the seeds are grown in the greenhouse and the mature plants are self-fertilized. Approximately 75000 seeds of the self-fertilized plants are grown in the greenhouse at 26 °C. As soon as the cotelydons can be observed above the ground, the seedlings are sprayed with 3 mM 2,6-diaminopurine during 10-11 days. Then the seedlings, whose development of the first pair of true leaves is not impaired, are selected. These seedlings are grown to maturity and examined for male sterility. The male sterile plants are crossed with a male fertile plant and the progeny is male fertile. Seedlings of self-fertilized plants of this progeny are tested for the percentage of male sterile plants. The male sterility and tolerance to purine analogues appears to be a monogenic recessive trait.

Analogous tests are run with tomato, chicory, endive, violet, melon, aubergine, impatiens, begonia, cyclamen and pelargonium. Similar male sterile plants are obtained and identified after foliar treatment with 3mM 2,6-diaminopurine.

## Claims

1. Plants selected from Solanaceae, Asteraceae, Violaceae Cucurbitaceae, Balsaminaceae, Begoniaceae, Geraniaceae and Primulaceae having homozygously or hetetozygously the tolerance gene against a purine analogue.

2. A plant according to Claim 1 of the Solanaceae.

3. A Solanaceae plant according to Claim 2, selected from pepper, tomato, aubergine, petunia and Schizanthus.

4. A plant according to Claim 3, which is
   a) Pepper
   or
   b) tomato.

5. A plant according to Claim 1 of the Asteraceae.

6. A plant according to Claim 5, which is
   a) endive
   or
   b) chicory.

7. A Violaceae plant according to Claim 1.

8. A Viola plant according to Claim 7.

9. A Cucurbitaceae plant according to Claim 1.

10. A melon plant according to Claim 9.

11. A plant according to Claim 1 selected from the family of the Balsaminaceae, Begoniaceae, Geraniaceae and Primulaceae.

12. A plant according to Claim 11 which is
    a) impatiens
    b) begonia
    c) geranium
    or
    d) cyclamen.

13. A plant according to Claims 1 to 12, having the tolerance gene against 2,6-diaminopurine.

14. A genic male sterile plant according to Claims 1 to 13 which is homozygous with respect to the tolerance to the purine analogue.

15. A method of selecting plants having homozygously the tolerance gene against a purine analogue, which comprises treating the seedlings during the development of the first pair of true leaves with a plant growth regulating amount of a purine analogue and selecting the plants which are not affected by such treatment.

16. The method of Claim 15, wherein the plant is of the family of the Solanaceae, Asteraceae, Violaceae, Cucurbitaceae, Balsaminaceae, Begoniaceae, Geraniaceae or Primulaceae.

17. The method of Claims 15 or 16 wherein the purine analogue is selected from 2,6-diaminopurine, 2-fluoroadenine and 8-azaadenine.

18. The method of Claims 15 to 17, comprising applying purine analogue concentrations from 0.1 to 15 mM till near the run off.

19. The method of Claim 18, wherein the spray application is repeated up to 15 consecutive days.

| | European Patent Office | **PARTIAL EUROPEAN SEARCH REPORT** which under Rule 45 of the European Patent Convention shall be considered, for the purposes of subsequent proceedings, as the European search report | Application Number |
|---|---|---|---|
| | | | EP 91 81 0491 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D A | PLANT PHYSIOLOGY, vol. 86, 1988, pages 1150-1154; & C.SOMERVILLE: "Positive selection for male sterile mutants of Arabidopsis lacking adenine phosphoribosyl transferase activity" * The whole document * | 1,2,5, 11,13- 17 | A 01 H 1/02 A 01 H 1/06 |
| Y | US-A-4 658 084 (GUELPH UNIVERSITY) * Abstract; column 4, line 35 - column 7, line 2; column 8, line 11 - column 12, line 42 * | 1,2,5, 11,13- 17 | |
| A | | 7,9 | |
| A | GB-A-2 208 346 (CROP RESEARCH DIV. DEPT. SCIENCE & IND.RESEARCH NW. ZEALAND) * Page 6, line 6 - page 24, line 13 * -/- | 1,2,5, 13-18 | |

|  | TECHNICAL FIELDS SEARCHED (Int. Cl.5) |
|---|---|
| | A 01 H |

### INCOMPLETE SEARCH

The Search Division considers that the present European patent application does not comply with the provisions of the European Patent Convention to such an extent that it is not possible to carry out a meaningful search into the state of the art of some of the claims

Claims searched completely : 1,2,5,7-9,11,13-19

Claims searched incompletely :

Claims not searched : 3,4,6,10,12

Reason for the limitation of the search:

Plant variety (See art. 53(b) of the European Patent Convention).

| Place of search THE HAGUE | Date of completion of the search 09-10-1991 | Examiner DISSEN H.D. |
|---|---|---|

**European Patent Office**

## PARTIAL EUROPEAN SEARCH REPORT

Application Number

EP 91 81 0491

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| A,D | PHYTOCHEMISTRY, vol. 25, no. 11, 1986, page 2445, & T.STUCHBURY: "Metabolism of purine nucleotides in the tomato plant"<br>--- | | |
| A | LEBARON & GRESSEL -eds- :"Herbicide resistance & Sons New York & P.S.CARLSON :"Herbicide resistance in plant cell cultures."<br>----- | | |
| | | | **TECHNICAL FIELDS SEARCHED (Int. Cl.5)** |

EPO FORM 1503 03.82 (P0410)